# EUROPEAN PATENT APPLICATION

(11) **EP 4 574 076 A1**
(43) Date of publication of application: **25.06.2025**
(21) Application number: 24201651.7
(22) Date of filing: 20.09.2024
(51) Int. Cl.: A61B 18/18, A61B 18/00

(54) **HAIR REMOVAL DEVICE**

(30) Priority: 21.09.2023 US 202363584225 P
(71) Applicant: Silkn Beauty Ltd, 3079516 Caesarea (IL)
(72) Inventor: MIDDENDORP, Oscar, Barendrecht (NL)
(74) Representative: Balder IP Law, S.L.

(57) **Abstract**

The invention is a handheld device that utilizes light for skin treatment. It consists of a handle with a power source, a user interface, and at least two capacitors. Connected to the top surface of the base unit is a head with at least two flash lamps and at least two reflectors. The device includes a control unit that regulates the voltage on the discharge capacitors to control the light energy from each flash lamp. Additionally, the device comes with an exchangeable cartridge, with each type of cartridge featuring a unique window layout and electronic chip containing information on lamp activation.

## Description

### BACKGROUND

### 1. TECHNICAL FIELD

The present invention relates to the field of hair removal devices, and more particularly, to a device using exchangeable cartridges accommodated for different treatment areas.

### 2. DISCUSSION OF RELATED ART

US patent 9827044 **incorporated by reference, disclose** applying galvanic current energy to skin at the area of a hair follicle. The galvanic current is generated by a galvanic micro-current unit. The galvanic current is a continuous wave or pulsed, without limitation, the galvanic current is in the range of 10-500 microamperes, the galvanic energy opens the pore around the hair follicle to increase exposure of the hair follicle for subsequent application of pulsed optical energy.

Pulsed optical energy is applied to the skin at the area of hair follicle so as to cause thermal destruction of the hair papilla. The pulsed optical energy is generated by a pulsed optical energy source which may emit intense pulsed light (IPL) and/or pulsed laser energy. The operating parameters may be as follows:
Optical energy-either IPL or laser-in a range of 2.5-40 Joules per cm2
Pulse duration of the optical energy-0.5-30 milliseconds
Spectrum for the laser-wavelength in a range of 700-1100 nm
Spectrum for the IPL-in a range of 450 nm-1200 nm

### BRIEF SUMMARY

The present invention discloses a handheld device for hair removal using light comprising:
a handle comprising:
   a power source;
   a user interface; and
   at least two capacitors; and
a head, connected to the top surface of the base unit, comprising:
   at least two flash lamps; and
at least two reflectors
   and
a control unit for controlling voltage on discharge capacitors which control light energy from each flash lamp.
exchangeable cartridge, wherein each type of cartridge has a different window layout and electronic chip including information of lamp activation.

The present invention discloses a handheld device for hair removal using light comprising:
a handle comprising:
   a power source.
   a user interface; and
   at least two capacitors; and
a head, connected to the top surface of the base unit, comprising:
   at least two flash lamps; and
   at least two reflectors
      and
   a control unit for controlling voltage on discharge capacitors which control light energy from each flash lamp.
   exchangeable cartridge, wherein each type of cartridge has a different window layout and electronic chip including information of lamp activation.

According to some embodiments of the present invention the exchangeable Cartridge is configured with a larger window size to accommodate the treatment protocol involving two lamps.

According to some embodiments of the present invention the exchangeable Cartridge is configured with a small window size to accommodate the treatment protocol involving a single lamp.

According to some embodiments of the present invention the handheld device further comprises a color sensor specifically designed to detect the user's skin color for determining the most appropriate radiation protocol for optimal treatment. According to some embodiments of the present invention the color sensor is activated at fixed intervals of applied pulses, the device ensures that the radiation protocols are accurately adjusted for different parts of the user's skin. According to some embodiments of the present invention the handheld device further comprising a frame electrode positioned along the boundary or edge of the cartridge, wherein the electrode is configured for delivering microcurrents to the skin, facilitating the opening of skin pores.

According to some embodiments of the present invention the frame comprises four round niches, specifically positioned to accommodate the placement of the four electrodes.

According to some embodiments of the present invention the handheld device further comprising electrical board which includes inner connectors and outer connector that establish connections with the sensors and electrodes within the cartridge unit, enabling the electrical board to connect with the treatment device, establishing the necessary electrical interface

According to some embodiments of the present invention the handheld device further comprising a Reflector frame comprised of four reflective walls, the reflector frame enhances the efficiency of the radiation or light transmission within the cartridge unit configured to direct and focus the emitted radiation towards the treatment area, maximizing the effectiveness of the device

According to some embodiments of the present invention each lamp is encapsulated by a distinct reflector configured to optimize the distribution and focus of the emitted radiation, wherein the reflectors are designed with a U shape, enabling them to effectively redirect and concentrate the emitted light towards the treatment area. According to some embodiments of the present invention the handheld further comprising a handle, equipped with two capacitors, configured to support the continuous activation of the lamps, sequential activation of the capacitors ensures a smooth and uninterrupted supply of energy to the lamps.

According to some embodiments of the present invention the control unit mange the ignition process by HV boost component which provides a high constant voltage during the ignition phase. micro current isolated driver. This driver is responsible for facilitating microcurrent functionality and features various components to ensure the protection of the main board from external voltage or current originating from the cartridge.

The present invention discloses a handheld device for hair removal treatment using light comprising:
a handle comprising:
   a power source.
   a user interface; and
   at least two capacitors; and
a head, connected to the top surface of the base unit, comprising:
   at least two flash lamps; and
at least two reflectors wherein each reflector shaped to have its reflection surface extending above the lamps, such the beam light of each lamp is not interfering with the light beam of the second lamp.
   and
a control unit for controlling voltage on discharge capacitors which control light energy from each flash lamp.

The present invention disclose a handheld device for hair removal using light comprising:
a handle comprising:
   a power source.
   a user interface; and
   at least one capacitors; and
a head, connected to the top surface of the base unit, comprising:
   at least two flash lamps; and
at least two reflectors
   and
a control unit for controlling voltage on discharge capacitor which control light energy from each flash lamp.
exchangeable cartridge, wherein each type of cartridge has a different window layout and electronic chip including information of lamp activation.

### BRIEF DESCRIPTION OF THE DRAWINGS

The present invention will be more readily understood from the detailed description of embodiments thereof made in conjunction with the accompanying drawings of which:
Figs. 1A, and 1B are overview illustrations of a skin care device and exchangeable cartridge units, according to some embodiments of the invention.
Figs. 2 is an exploded view illustration of a head of exchangeable cartridge unit, according to some embodiments of the invention.
Fig. 3 is an exploded view illustration of a hair removal device, showing the lamps and reflectors according to some embodiments of the invention.
Figs. 4 is an illustration of hair removal device interior design showing the lamps and reflectors, according to some embodiments of the invention.
Fig. 5 is an illustration of a hair removal device's upper part, showing the reflectors, Fan, LEDs and button according to some embodiments of the invention.
Fig. 6 is an illustration of a hair removal device's front panel, showing the reflectors according to some embodiments of the invention.
Figs. 7 is an illustration of a hair removal device handle, showing the capacitors, according to some embodiments of the invention.
Figs. 8 is a blocking diagram, showing the main board cartridge board and trigger board, according to some embodiments of the invention.
Figs. 9 is a block diagram, showing the main board: controller, lamps, chargers, according to some embodiments of the invention;
Figs. 10 is a block diagram, showing the main board: logic optic isolation and microcurrent circuit, according to some embodiments of the invention;
Figs. 11 is a block diagram, showing the electronic board of the cartridge unit, according to some embodiments of the invention;
Figs. 12 is a block diagram, showing the electronic harness of the HV capacitor, according to some embodiments of the invention;
Figs. 13 is a block diagram, showing the electronic board of the trigger button, according to some embodiments of the invention;
Figs. 14 is a block diagram, showing the electronic board of the input power supply, MMI LEDs and on/off switch, according to some embodiments of the invention;
Figs. 15 is a block diagram, showing the electronic board of the trigger button and Fan circuit, according to some embodiments of the invention;
Figs. 16A is an illustration of hair removal device in different rotation state, according to some embodiments of the invention.
Figs. 16B is an illustration of a hair removal device in a different rotation state, according to some embodiments of the invention.
Figs. 16C is illustration of hair removal device in different rotation state, according to some embodiments of the invention.
Figs. 16D is an illustration of a hair removal device in a different rotation state, according to some embodiments of the invention.
Figs. 16E is an illustration of a hair removal device in different rotation state, according to some embodiments of the invention.
Figs. 16F is an illustration of a hair removal device in a different rotation state, according to some embodiments of the invention.
Figs. 16G is an illustration of a hair removal device in a different rotation state, according to some embodiments of the invention.

### DETAILED DESCRIPTION

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present invention discloses a hand-held device contains Xenon lamp, which provides the electro-optic power for the hair removal treatment and using galvanic current energy to apply the micro current on the treatment area.

The present invention pertains to a handheld device designed for hair removal utilizing light technology. The device incorporates a handle, which houses essential components such as a power source, a user interface, and at least two capacitors. The capacitors serve a vital role in storing and discharging electrical energy required for the operation of the device.

Connected to the handle is a head unit that is responsible for delivering light-based hair removal treatment. This head unit comprises at least two flash lamps, which emit visible wavelengths of light. The emitted light is then directed and focused by at least two reflectors, optimizing its efficiency and targeting specific areas of the skin in uniform power distribution.

To ensure precise control over the light energy emitted by each flash lamp, the device incorporates a control unit. This control unit regulates the voltage applied to the discharge capacitors, effectively managing the intensity and duration of the light emitted by the flash lamps. This allows for customizable treatment protocols based on the user's specific needs.

Furthermore, the device incorporates an exchangeable cartridge system. Each cartridge type is designed with a different window layout, providing versatility in addressing various treatment areas and concerns. Additionally, each cartridge is equipped with an electronic chip that contains information related to lamp activation. This chip facilitates seamless integration with the device, enabling automatic recognition and configuration of the appropriate treatment settings.

In summary, the described handheld device offers a user-friendly and customizable solution for skin treatment using light technology. The combination of the handle, head unit, capacitors, reflectors, and control unit, along with the exchangeable cartridge system, ensures effective and tailored treatment results for hair removal and treatment areas.

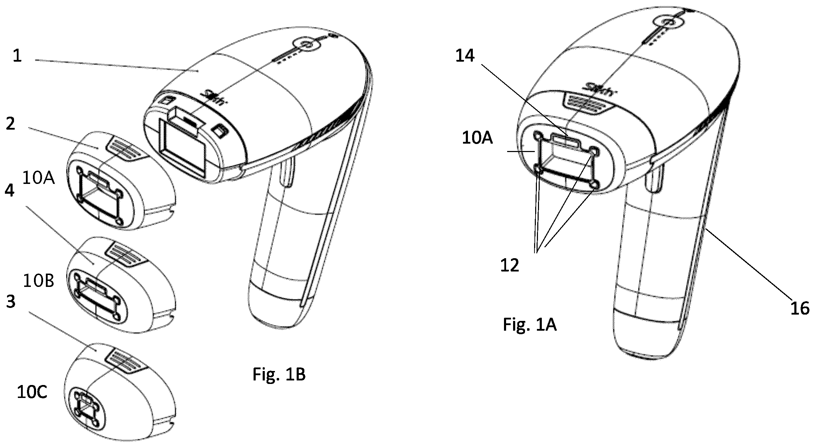

Figs. 1A, and 1B are overview illustrations of a skin care device and exchangeable cartridge units, according to some embodiments of the invention.

The handheld hair removal treatment device, designated as Device 1, is composed of three main components: the body (1), the handle (16), and the exchangeable cartridge (2, 3, or 4), as described in fig. 1A and 1B. Each exchangeable cartridge is designed with distinct layouts, featuring varying sizes and shapes for their respective windows. Specifically, exchangeable Cartridge 2 is configured with a larger window size to accommodate the treatment protocol involving two lamps. This design allows for a broader coverage area during the skin treatment process, ensuring uniform distribution of energy resulting in efficient and effective results.

On the other hand, exchangeable Cartridge 3 is designed with a smaller window size, tailored to support the treatment protocol operating/involving a single lamp. This configuration is ideal for targeting medium size treatment areas on the skin. Furthermore, there is another variation of exchangeable Cartridge 4, which features a smaller window size as well. However, this particular configuration is optimized for treating small areas, providing precise and concentrated treatment for localized skin concerns.

By offering exchangeable cartridges with different window sizes and shapes, Device 1 provides versatility in addressing various hair removal. Users can select the appropriate cartridge based on their specific requirements, allowing for personalized and targeted hair removal routines.

Each of the exchangeable cartridges integrated into the handheld skin treatment device is equipped with a color sensor (14) specifically designed to detect the user's skin color. This innovative feature plays a crucial role in determining the most appropriate radiation protocol for optimal treatment.

By activating the color sensor at fixed intervals of applied pulses, the device ensures that the radiation protocols are accurately adjusted for different parts of the user's skin. This dynamic adjustment is essential because various areas of the skin may have different color tones and characteristics.

The color sensor detects the user's skin color by analyzing the reflected light from the skin surface. This information is then utilized by the device's algorithm to determine the most suitable radiation protocol for the detected skin color. This intelligent system allows for personalized treatment, ensuring that the device delivers the appropriate level of radiation for each specific skin type.

By periodically activating the color sensor, the handheld device continuously assesses the user's skin color and adjusts the radiation protocol accordingly. This ensures that the treatment remains safe, effective, and tailored to the individual's unique needs. Whether treating different areas of the body or accommodating changes in the user's skin tone over time, the color sensor provides an essential feature that enhances the precision and accuracy of the hair removal process.

In Automatic Mode, in every pulse (optionally every predefined number of pulses), skin color sensor reports the color of the skin, the device sets the energy level skin color to determine the appropriate radiation protocol.

In Manual Mode, a skin color sensor reports the color of the skin when skin contact is detected.

Incorporated within all exchangeable cartridges is a frame electrode (25) positioned along the boundary or edge of the cartridge. This electrode serves a crucial function by delivering microcurrents to the skin, facilitating the opening of skin pores. By employing this mechanism, the handheld skin treatment device enhances the effectiveness of the treatment process.

The frame electrode emits gentle microcurrents that stimulate the skin's surface, promoting the dilation of pores.

The microcurrents emitted by the frame electrode are carefully calibrated to ensure they remain at a safe and comfortable level for the user. The treatment process becomes a pleasant experience as the microcurrents contribute to a gentle and soothing sensation on the skin.

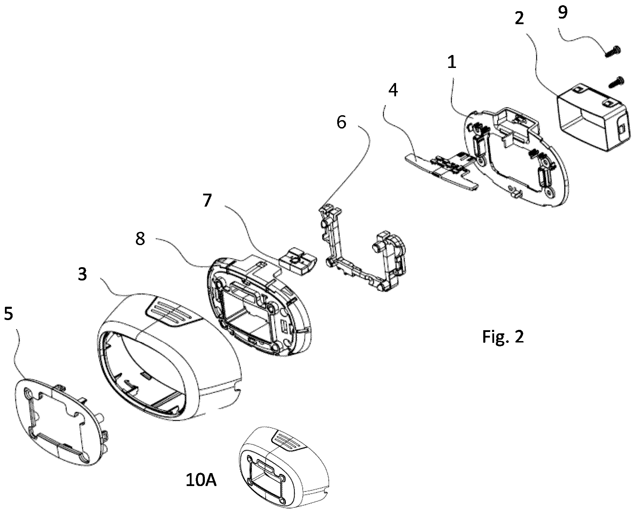

Figs. 2 is an exploded view illustration of a head of exchangeable cartridge unit, according to some embodiments of the invention.

The head of the exchangeable cartridge unit 10A (as seen in fig. 2) consists of several components that work together to ensure its functionality. These components include:
1. Front panel 5: This panel features a rectangular window that allows the transmission of radiation or light during the treatment process. It is designed with four round niches, specifically positioned to accommodate the placement of the four electrodes (12). Additionally, a smaller upper niche is provided to house the color sensor, which detects the user's skin color.
2. Case 3: The case serves as the outer enclosure, encompassing and protecting all the internal parts of the cartridge unit. It provides structural support and ensures the integrity of the components within.
3. Inner frame 8: The inner frame acts as a structural component within the cartridge unit, providing stability and support to the various internal elements.
4. Electrical frame 6: This frame is responsible for hosting the four electrodes located at each corner of the cartridge unit. The lower electrodes are connected via a connector to the upper contacts, facilitating the flow of electrical signals.
5. Electrical board 4: The electrical board includes inner connectors that establish connections with the sensors and electrodes within the cartridge unit. It also features an outer connector that enables the electrical board to connect with the treatment device, establishing the necessary electrical interface.
6. Back frame 1: The back frame completes the structural integrity of the cartridge unit, providing stability and support to the various components.
7. Reflector frame 2: Comprised of four reflective walls, the reflector frame enhances the efficiency of the radiation or light transmission within the cartridge unit. It helps to direct and focus the emitted radiation towards the treatment area, maximizing the effectiveness of the device.

Together, these components form the head of the exchangeable cartridge unit, each playing a vital role in ensuring the proper functioning and performance of the handheld skin treatment device.

The cartridge electrical connector features four contacts electrodes (12) which are specifically designated for the purpose of contact detection. This contact detection mechanism is implemented on the frame of the window using a conducting element.

The primary objective of this contact-electrode configuration is to ensure that the device remains inactive unless all four electrodes establish contact with the user's skin simultaneously. By requiring signals from all four electrodes, the system effectively prevents any possibility of radiation leakage.

This safety measure guarantees that the device remains non-operational until the user's skin makes proper contact with all four electrodes, thereby minimizing the risk of any potential radiation exposure.

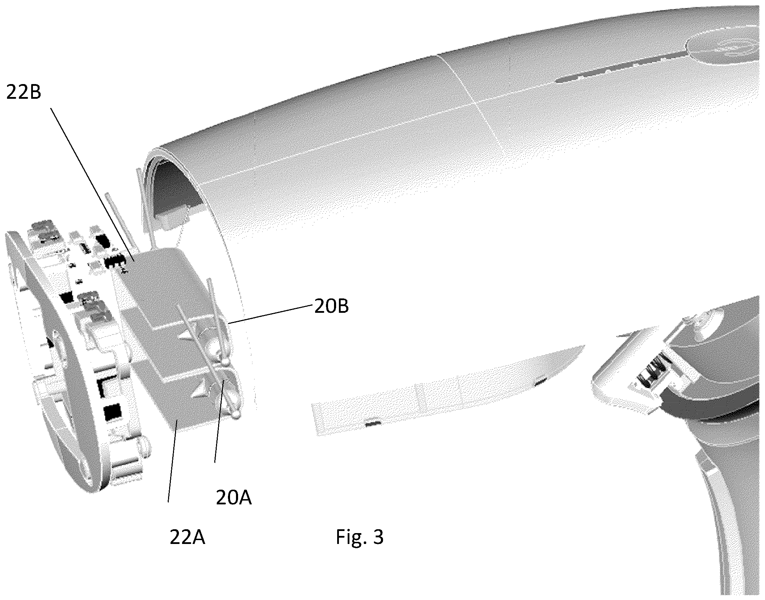

Fig. 3 is an exploded view illustration of a hair removal device, showing the lamps and reflectors according to some embodiments of the invention.

Located within the front body of the device, there are two lamps, namely Lamp 20A and Lamp 20B. These lamps play a central role in the functionality of the device, providing the necessary radiation or light for the hair removal treatment process.

To optimize the distribution and focus of the emitted radiation, each lamp is encapsulated by a distinct reflector. Reflector 22A and Reflector 22B are specifically designed with a U shape, enabling them to effectively redirect and concentrate the emitted light towards the treatment area.

The U-shaped reflectors are strategically positioned around each lamp, encompassing and surrounding them. This configuration ensures that the emitted energy is efficiently directed towards the desired target area of the skin. By shaping the reflectors in a U shape, the device can achieve a more precise and controlled distribution of the energy, maximizing the effectiveness of the treatment.

The reflectors not only enhance the efficiency of the radiation but also serve as protective barriers, preventing the direct exposure of the lamps and minimizing the risk of accidental contact or damage.

By utilizing distinct reflectors for each lamp and their U-shaped design, the handheld skin treatment device optimizes the delivery and distribution of energy, providing targeted and effective treatment to the desired skin areas.

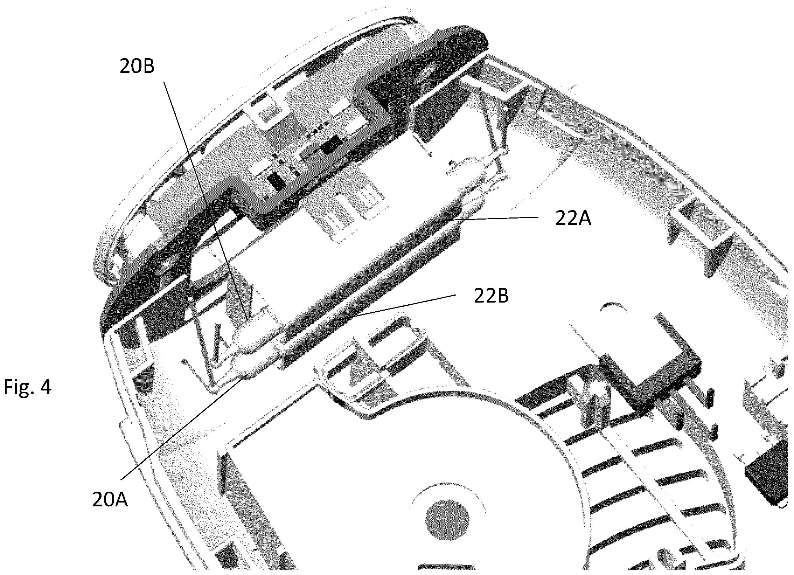

Figs. 4 is an illustration of hair removal device interior design showing the lamps and reflectors, according to some embodiments of the invention.

Within the front body of the device are installed two lamps 20A and 20B, wherein each lamp is encapsulated by different reflectors 22A, 22B.

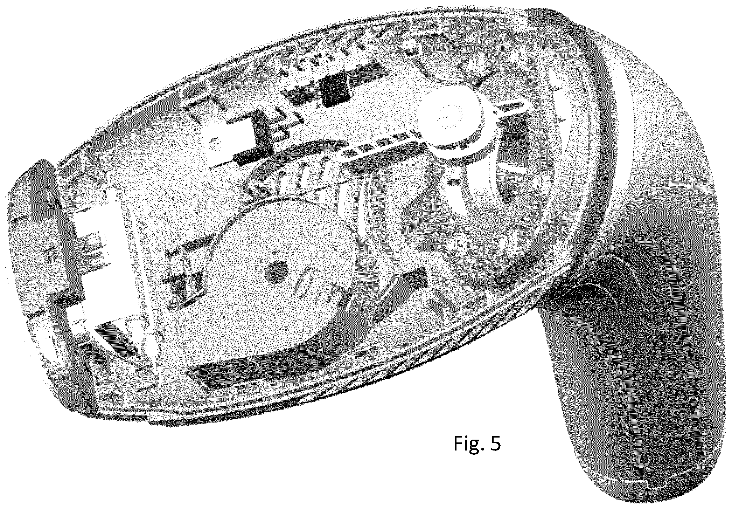

Fig. 5 is an illustration of a hair removal device's upper part, showing the reflectors, Fan, LEDS and button according to some embodiments of the invention.

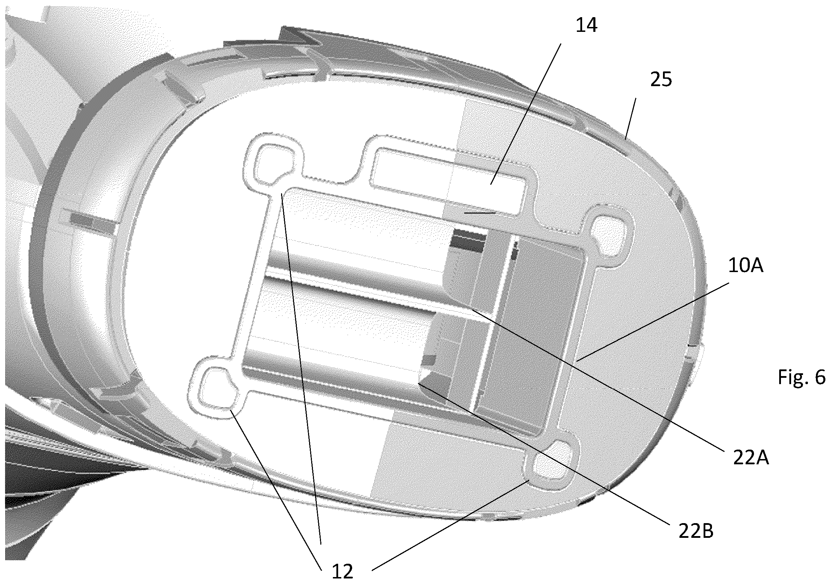

Fig. 6 is an illustration of a hair removal device's front panel, showing the reflectors according to some embodiments of the invention.

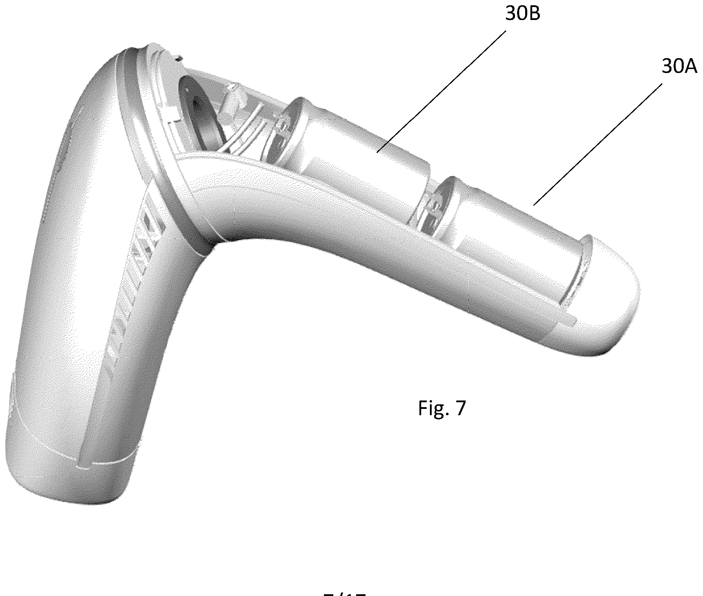

Figs. 7 is an illustration of a hair removal device handle, showing the capacitors, according to some embodiments of the invention.

The device handle is equipped with two capacitors, labeled Capacitor 30A and Capacitor 30B. These capacitors play a crucial role in the operation of the device, specifically in supporting the continuous activation of the lamps.

According to some embodiments handle is equipped with one capacitor.

Controlled by the electric board, the capacitors are utilized to overcome the time delays typically associated with charging and discharging processes. The sequential activation of the capacitors ensures a smooth and uninterrupted supply of energy to the lamps.

By employing this sequential activation mechanism, the device can maintain a consistent and steady output of radiation or light, without any noticeable interruptions or fluctuations. This is particularly important in achieving effective hair removal treatment results, as a continuous and stable energy supply is required.

The electric board manages and regulates the activation of the capacitors, allowing for precise control over the energy levels delivered to the lamps. This control enables the device to offer at least five distinct energy levels, providing flexibility and customization in the treatment process. Users can select the desired energy level based on their specific needs, ensuring optimal treatment outcomes.

Overall, the incorporation of capacitors and their controlled activation within the device handle ensures a seamless and efficient operation, enabling continuous activation of the lamps and delivering consistent energy levels for effective hair removal treatment.

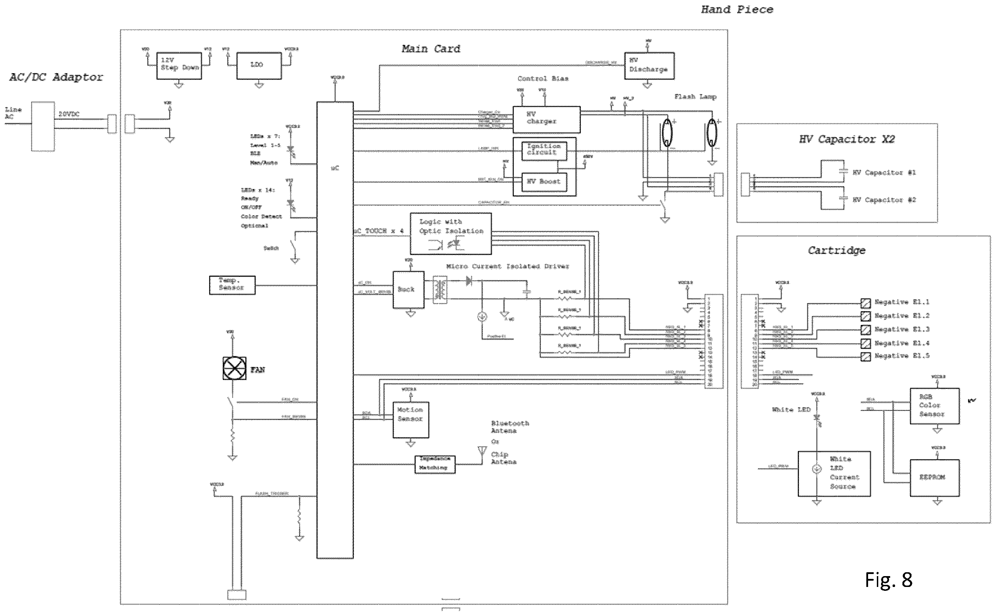

Figs. 8 is a blocking diagram, showing the main board cartridge board and trigger board, according to some embodiments of the invention.

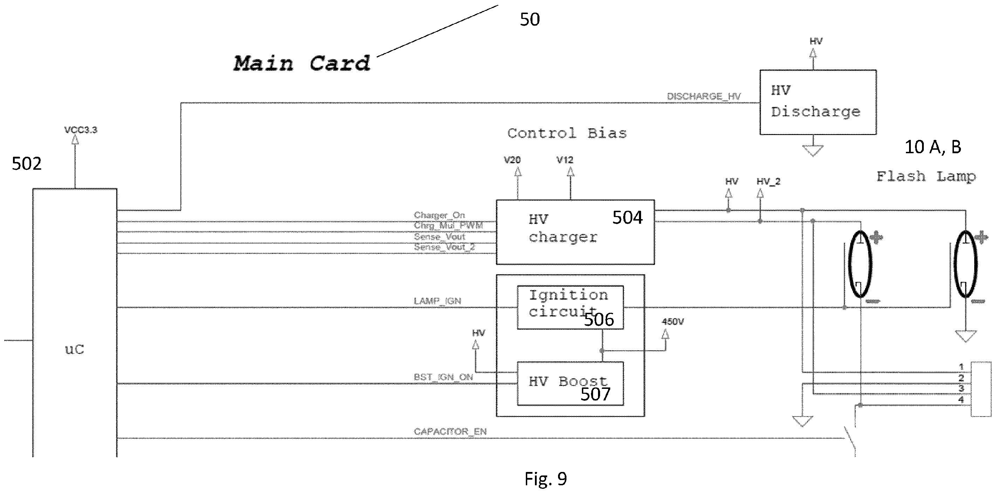

Figs. 9-16 are block diagrams, showing different sections of the main board and cartridge electronical configuration: controller, lamps, chargers, according to some embodiments of the invention.

Figure 9 illustrates the first section of the main board, which encompasses essential components responsible for the device's operation. These components include the main controller (502), HV charger (504), ignition circuit (506), and HV boost (507).

The main controller (502) serves as the central component of the board, responsible for managing and coordinating the device's overall functionality. It controls various operations, such as regulating energy levels, monitoring safety features, and facilitating communication between different parts of the device.

The HV charger (504) plays a crucial role in enabling the quick discharge of the capacitor. This component ensures that the capacitor can rapidly release stored energy when needed, allowing for efficient and timely activation of the device's functions.

The ignition circuit (506) is specifically designed to initiate the ignition process of the flash lamps (20A, 20B). It achieves this by ionizing the lamp wire, creating a conductive path that facilitates the discharge of the stored energy. The ignition circuit's configuration ensures that the flash lamps can be promptly ignited when the device is activated.

To facilitate the ignition process, the HV boost (507) component provides a high constant voltage during the ignition phase. This high voltage is crucial for initiating the discharge of the capacitor, overcoming the initial resistance of the flash lamps, and ensuring a consistent and reliable ignition of the lamps.

By incorporating these components and their specific configurations, the main board effectively manages the activation and operation of the device. The main controller orchestrates the device's functions, while the HV charger, ignition circuit, and HV boost work together to facilitate quick discharge, ignition, and maintenance of the necessary energy levels for optimal performance.

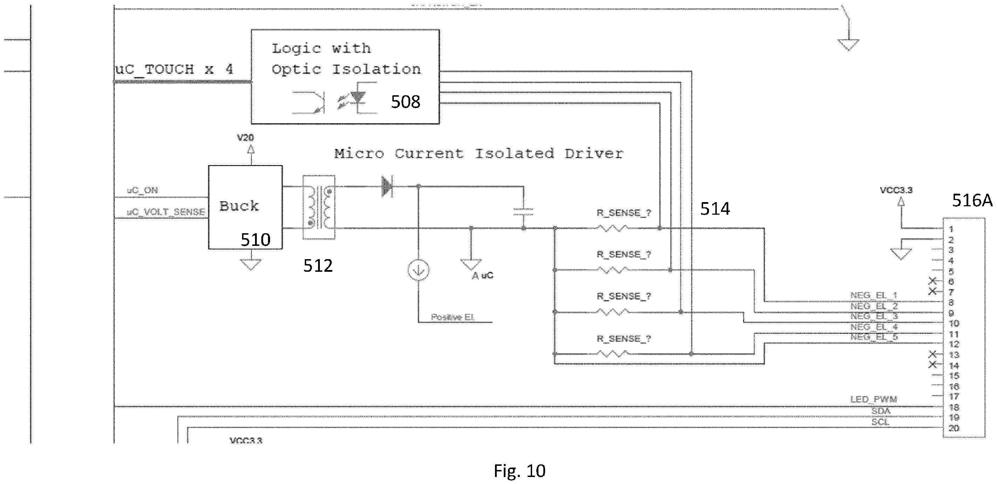

Figure 10 represents a blocking diagram that illustrates the second section of the main board, specifically the micro current isolated driver. This driver is responsible for facilitating microcurrent functionality and features various components to ensure the protection of the main board from external voltage or current originating from the cartridge.

The key component of the micro current isolated driver is the microcurrent optic isolation, which is designed to safeguard the main board. This isolation mechanism prevents any potentially harmful voltage or current from entering the main board, offering protection in accordance with certain embodiments of the invention. Microcurrent optic isolation comprises several elements to fulfill its protective function.

These include:
Logic optic isolation (508): This component establishes an optical barrier, ensuring complete isolation between the microcurrent driver and the main board. It prevents any electrical signals or interference from passing through, maintaining the integrity and safety of the main board.

Buck converter (510): The buck converter is responsible for converting the voltage levels required for the microcurrent functionality. It efficiently steps down the voltage from the power source to the appropriate levels needed to drive the microcurrent driver.

R-sense (514): R-sense is a sensing element that helps monitor and regulate the current flowing through the microcurrent driver. It aids in ensuring that the microcurrents are accurately maintained within the desired range.

Contacts (516A): The contacts are connectors that establish the necessary electrical connections between the microcurrent driver and the cartridge. These contacts facilitate the transmission of signals and enable the microcurrent functionality to be applied effectively during the hair removal treatment process.

Together, these components form the microcurrent isolated driver of the main board. By employing microcurrent optic isolation and its associated elements, the driver provides essential protection for the main board, enabling the safe and reliable operation of the device's microcurrent functionality.

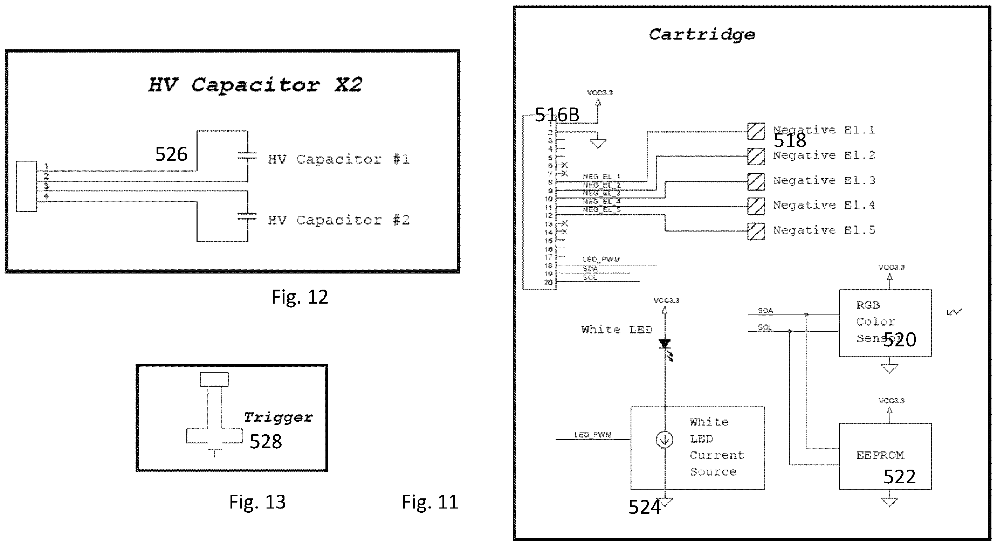

Fig. 11 is a block diagram, showing the electronic board of the cartridge unit, according to some embodiments of the invention.

The cartridge unit is comprised of contact 516B connected to the contacts 516A at the main board, RG color sensor 520 configured to identify skin color, EEPROM 522, and white LED current source and five electrodes 518.

Contact 516B: This contact serves as a connection point within the cartridge unit and is connected to the contacts 516A located on the main board. This connection allows for the transfer of signals and power between the cartridge unit and the main board. White LED current source: This component acts as a source of electrical current for the white LEDs integrated into the cartridge unit. The white LEDs emit light that is used during the treatment process.

Five electrodes 518: The five electrodes are strategically positioned within the cartridge unit. These electrodes facilitate the delivery of electrical signals or microcurrents to the user's skin during the treatment. They are responsible for various functions such as stimulation, opening skin pores, or enhancing the effectiveness of the treatment.

RG color sensor 520: The RG color sensor plays a vital role in identifying the user's skin color. By detecting the levels of red (R) and green (G) light reflected from the skin, the color sensor accurately determines the skin colon tone. This information is essential for selecting the appropriate treatment parameters and protocols.

Together, these components within the cartridge unit enable various functionalities such as skin color detection, light emission, electrical stimulation, and treatment customization. The integration of these elements ensures a comprehensive and tailored skin treatment experience for the user.

Figure 12 presents a block diagram showcasing the HV (high voltage) capacitor of the electronic board, as per specific embodiments of the invention. This HV capacitor, labeled as X2 526, is composed of two primary components: HV capacitor 1 and HV capacitor 2.

HV capacitor 1 and 2: These components are designed to store and discharge high-voltage electrical energy efficiently. It plays a crucial role in the overall functioning of the electronic board, particularly in applications that require the utilization of high-voltage power.

By combining HV capacitor 1 and HV capacitor 2, the X2 526 unit ensures the availability of sufficient high-voltage energy when required. These capacitors work in tandem to provide the necessary electrical power to various components and circuits on the electronic board, supporting their proper functionality.

Figs. 13 is a blocking diagram, showing the electronic board of the trigger button 528 according to some embodiments of the invention.

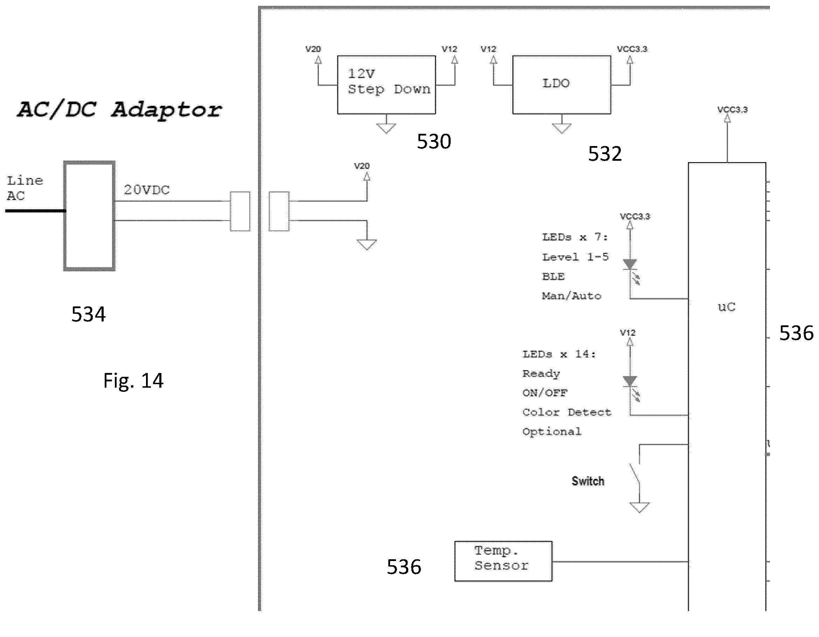

Fig. 14 is a blocking diagram, showing the third part of the main electronic board according to some embodiments of the invention. This part of the electronic board comprises: the temperature sensor 536.

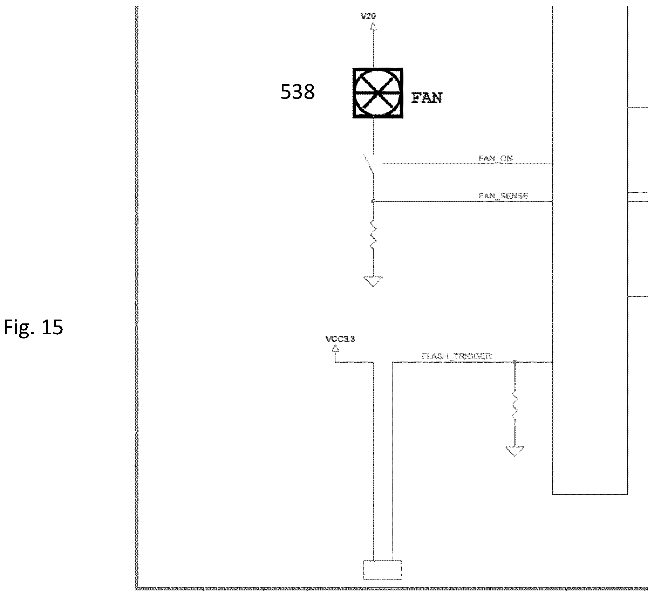

Fig. 15 is a blocking diagram, showing the fourth part of the main electronic board, according to some embodiments of the invention. This part of the electronic board comprises fan 538.

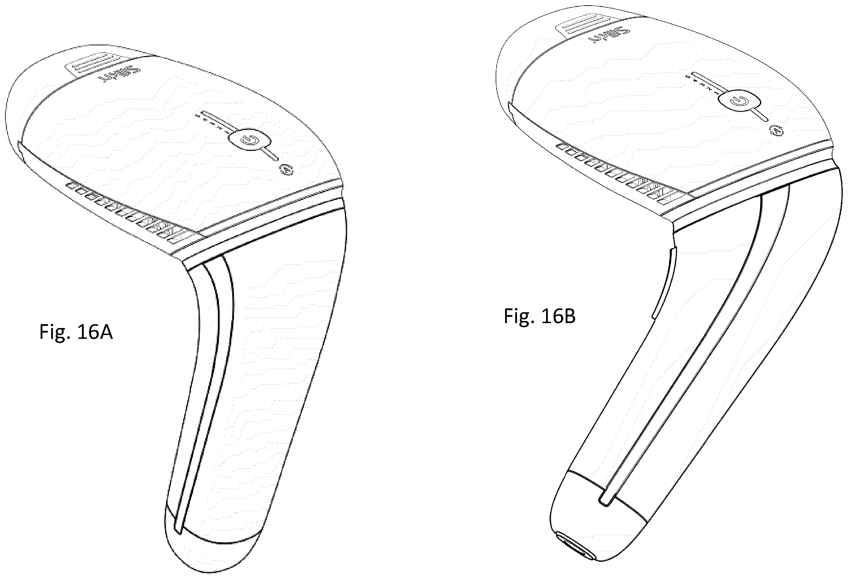

Figures 16A-16G provide a series of illustrations depicting the hair removal device in various rotation states. These visuals effectively demonstrate the flexibility and versatility of the device, showcasing its ability to accommodate different areas of the body with ease and efficiency.

The device's design allows for easy manipulation and rotation, ensuring optimal usability for each specific part of the body. By adjusting the orientation of the device, users can adapt it to different angles and contours, facilitating comfortable and effective treatment on various body areas.

The illustrations highlight the device's adaptability, showcasing how it can be effortlessly rotated and positioned to cater to specific treatment requirements. Whether targeting larger or smaller areas, flat or curved surfaces, the device can be adjusted accordingly, offering enhanced convenience and user-friendliness.

This flexibility in rotation enables users to access hard-to-reach areas, such as the back, shoulders, or neck, without straining or compromising the treatment process. It allows for seamless treatment application across different body parts, ensuring consistent results throughout.

Overall, the illustrations in Figures 16A-16G emphasize the device's capacity to adapt to the body's contours, enabling efficient and convenient usage across various areas. This versatility enhances user experience, making the device a practical and adaptable solution for comprehensive hair removal treatments.

Figs. 16A is illustration of hair removal device in different rotation state, according to some embodiments of the invention.

Figs. 16B is an illustration of a hair removal device in a different rotation state, according to some embodiments of the invention.

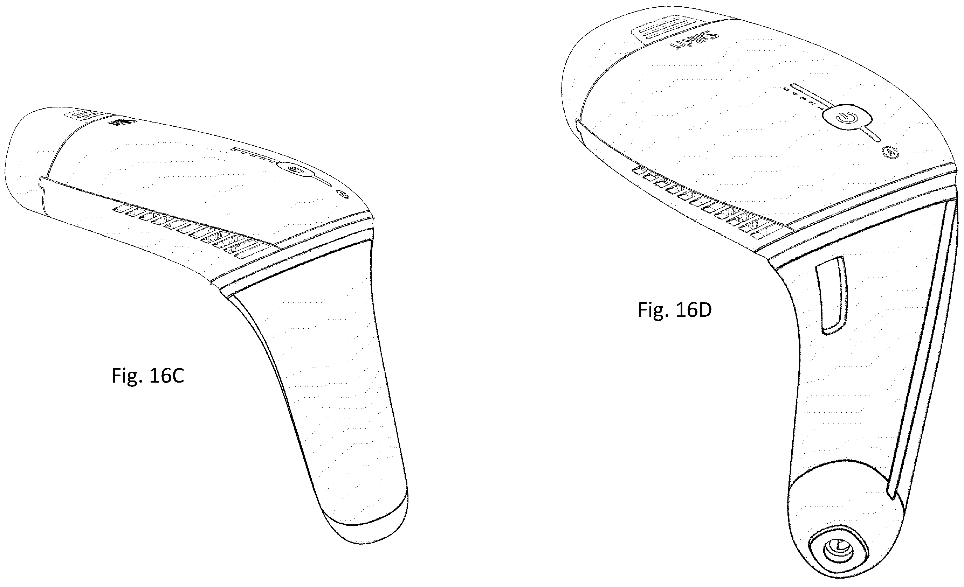

Figs. 16C is illustration of hair removal device in different rotation state, according to some embodiments of the invention.

Figs. 16D is an illustration of a hair removal device in a different rotation state, according to some embodiments of the invention.

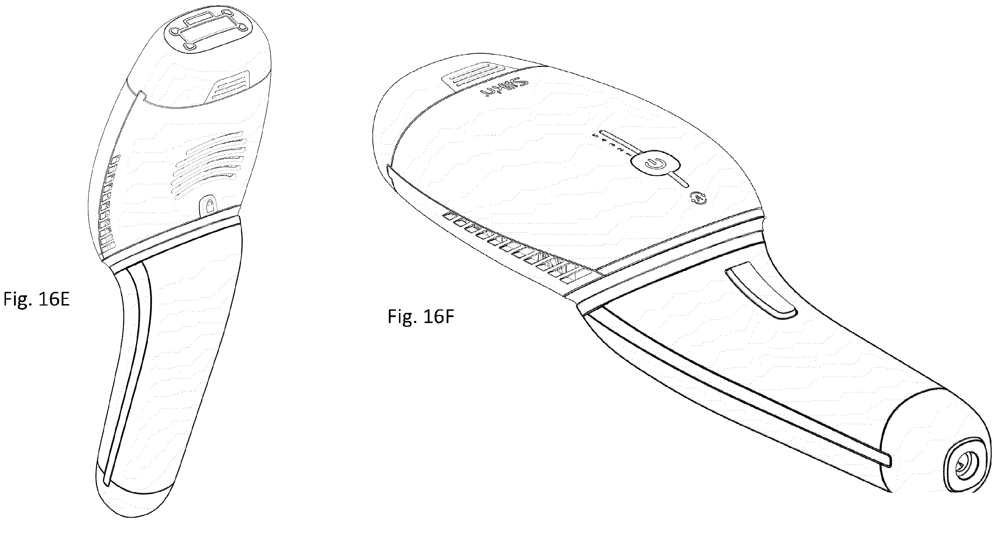

Figs. 16E is an illustration of a hair removal device in different rotation state, according to some embodiments of the invention.

Figs. 16F is an illustration of a hair removal device in a different rotation state, according to some embodiments of the invention.

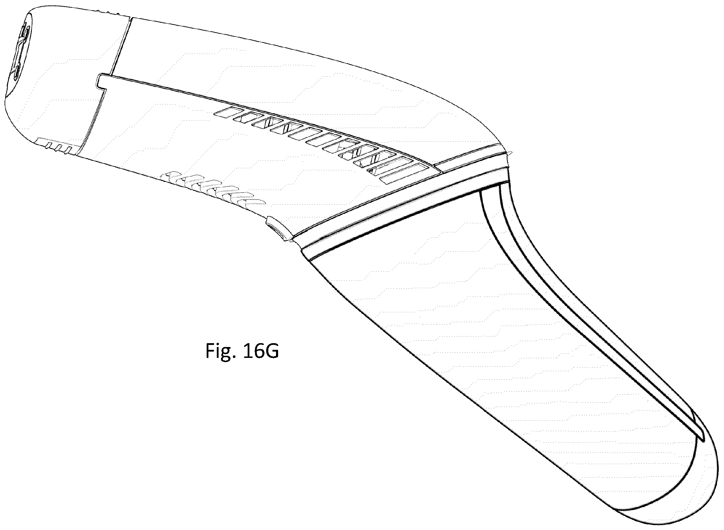

Figs. 16G is an illustration of a hair removal device in a different rotation state, according to some embodiments of the invention.

According to specific embodiments of the present invention, the pulse sequence process is outlined as follows:
- The sequence is initiated when the skin sensor detects skin contact, requiring at least three touch sensors to be in contact with the skin. This ensures reliable and accurate detection of skin contact.
- Upon skin contact detection, the "Ready" LED is activated and enters a blinking state. This visual indicator informs the user that the device is ready for operation.
- The device identifies when the user presses the trigger button, signaling their intention to begin the treatment process.
- Microcurrent pulses are released by the electrodes, providing the desired stimulation or treatment to the skin.
- In Automatic Mode, before every pulse, the skin color sensor reports the color of the skin. This information is used to dynamically adjust the energy level settings of the device, ensuring an appropriate treatment intensity. The energy level is determined according to section 6.1.1 of the device's specifications.
- In Manual Mode, the skin color sensor reports the color of the skin upon initial skin contact. This provides valuable information for setting the appropriate energy level for the treatment.

The charging circuit discharges through the lamp if the trigger button is pressed while the device is in contact with the skin, the skin color is lighter than the level VI threshold, and the charging circuit is fully energized. This feature optimizes the discharge process and ensures the correct energy level is applied based on the skin color and charging status.

If there is a failure to flash, the device addresses the issue by reattempting the flash sequence after a 200 ms delay. This allows for a retry without a microcurrent pulse to correct any potential technical or operational issues.

The flash sequence will continue to repeat as long as skin contact is maintained, and the measured skin color remains lighter than the level VI threshold. The skin color is measured every 20th flash to monitor any changes or adjustments required throughout the treatment.

By following this pulse sequence process, the device ensures accurate detection, appropriate energy settings, and reliable treatment outcomes, taking into account the user's skin colon and treatment preferences.

In the above description, an embodiment is an example or implementation of the inventions. The various appearances of "one embodiment," "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

It is to be understood that the phraseology and terminology employed herein is not to be construed as limiting and are for descriptive purposes only.

The principles and uses of the teachings of the present invention may be better understood with reference to the accompanying description, figures and examples.

It is to be understood that the details set forth herein do not construe a limitation to an application of the invention.

Furthermore, it is to be understood that the invention can be carried out or practiced in various ways and that the invention can be implemented in embodiments other than the ones outlined in the description above.

It is to be understood that the terms "including", "comprising", "consisting of" and grammatical variants thereof do not preclude the addition of one or more components, features, steps, or integers or groups thereof and that the terms are to be construed as specifying components, features, steps or integers.

If the specification or claims refer to "an additional" element, that does not preclude there being more than one of the additional elements.

It is to be understood that where the claims or specification refer to "a" or "an" element, such reference is not construed that there is only one of that elements.

It is to be understood that where the specification states that a component, feature, structure, or characteristic "may", "might", "can" or "could" be included, that particular component, feature, structure, or characteristic is not required to be included.

Where applicable, although state diagrams, flow diagrams or both may be used to describe embodiments, the invention is not limited to those diagrams or to the corresponding descriptions. For example, flow need not move through each illustrated box or state, or in exactly the same order as illustrated and described.

Methods of the present invention may be implemented by performing or completing manually, automatically, or a combination thereof, selected steps or tasks.

The term "method" may refer to manners, means, techniques and procedures for accomplishing a given task including, but not limited to, those manners, means, techniques and procedures either known to, or readily developed from known manners, means, techniques and procedures by practitioners of the art to which the invention belongs.

The descriptions, examples, methods and materials presented in the claims and the specification are not to be construed as limiting but rather as illustrative only.

Meanings of technical and scientific terms used herein are to be commonly understood as by one of ordinary skill in the art to which the invention belongs, unless otherwise defined.

The present invention may be implemented in the testing or practice with methods and materials equivalent or similar to those described herein.

Any publications, including patents, patent applications and articles, referenced or mentioned in this specification are herein incorporated in their entirety into the specification, to the same extent as if each individual publication was specifically and individually indicated to be incorporated herein. In addition, citation or identification of any reference in the description of some embodiments of the invention shall not be construed as an admission that such reference is available as prior art to the present invention.

While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention should not be limited by what has thus far been described, but by the appended claims and their legal equivalents.

## Claims

1. A handheld device for hair removal using light comprising:
a handle comprising:
a power source.
a user interface;
at least two capacitors;
a head, connected to the top surface of the base unit, comprising:
at least two flash lamps;
at least two reflectors ; and
a control unit for controlling voltage on discharge capacitors which control light energy from each flash lamp.
exchangeable cartridge, wherein each type of cartridge has a different window layout and electronic chip including information of lamp activation.

2. The handheld device of claim 1 wherein the exchangeable Cartridge is configured with a larger window size to accommodate the treatment protocol involving two lamps.

3. The handheld device of claim 1 wherein the exchangeable Cartridge is configured with a small window size to accommodate the treatment protocol involving a single lamp.

4. The handheld device of claim 1 further comprising a color sensor specifically designed to detect the user's skin color for determining the most appropriate radiation protocol for optimal treatment.

5. The handheld device of claim 4 wherein the color sensor is activated at fixed intervals of applied pulses, the device ensures that the radiation protocols are accurately adjusted for different parts of the user's skin.

6. The handheld device of claim 1 further comprising a frame electrode positioned along the boundary or edge of the cartridge, wherein the electrode is configured for delivering microcurrents to the skin, facilitating the opening of skin pores.

7. The handheld device of claim 1 wherein the frame comprises four round niches, specifically positioned to accommodate the placement of the four electrodes.

8. The handheld device of claim 1 further comprising electrical board which includes inner connectors and outer connector that establish connections with the sensors and electrodes within the cartridge unit, enabling the electrical board to connect with the treatment device, establishing the necessary electrical interface

9. The handheld device of claim 1 further comprising a Reflector frame comprised of four reflective walls, the reflector frame enhances the efficiency of the radiation or light transmission within the cartridge unit configured to direct and focus the emitted radiation towards the treatment area, maximizing the effectiveness of the device

10. The handheld device of claim 1 wherein each lamp is encapsulated by a distinct reflector configured to optimize the distribution and focus of the emitted radiation, wherein the reflectors are designed with a U shape, enabling them to effectively redirect and concentrate the emitted light towards the treatment area.

11. The handheld device of claim 1 further comprising a handle, equipped with two capacitors, configured to support the continuous activation of the lamps, sequential activation of the capacitors ensures a smooth and uninterrupted supply of energy to the lamps.

12. The handheld device of claim 1 wherein the control unit mange the ignition process by HV boost component which provides a high constant voltage during the ignition phase. micro current isolated driver. This driver is responsible for facilitating microcurrent functionality and features various components to ensure the protection of the main board from external voltage or current originating from the cartridge.

13. A handheld device for hair removal treatment using light comprising:
a handle comprising:
a power source.
a user interface;
at least two capacitors;
a head, connected to the top surface of the base unit, comprising:
at least two flash lamps;
at least two reflectors wherein each reflector shaped to have its reflection surface extending above the lamps, such the beam light of each lamp is not interfering with the light beam of the second lamp; and
a control unit for controlling voltage on discharge capacitors which control light energy from each flash lamp.

14. A handheld device for hair removal using light comprising:
a handle comprising:
a power source.
a user interface; and
at least one capacitors; and
a head, connected to the top surface of the base unit, comprising:
at least two flash lamps; and
at least two reflectors
and
a control unit for controlling voltage on discharge capacitor which control light energy from each flash lamp.
exchangeable cartridge, wherein each type of cartridge has a different window layout and electronic chip including information of lamp activation.
